# EUROPEAN PATENT SPECIFICATION

(11) **EP 3 241 502 B1**
(45) Date of publication and mention of the grant of the patent: **07.06.2023**
(21) Application number: 15875261.8
(22) Date of filing: 30.12.2015
(51) Int. Cl.: A61B 17/072, A61B 90/00, A61B 17/00

(54) **STAPLING HEAD ASSEMBLY AND SUTURING AND CUTTING APPARATUS FOR ENDOSCOPIC SURGERY**
KLAMMERKOPFANORDNUNG SOWIE NÄH- UND SCHNEIDEVORRICHTUNG FÜR DIE ENDOSKOPISCHE CHIRURGIE
SYSTÈME DE TÊTE D'AGRAFAGE ET APPAREIL DE SUTURE ET DE COUPE POUR LA CHIRURGIE ENDOSCOPIQUE

(30) Priority: 30.12.2014 CN 201410843332; 30.12.2014 CN 201420858544 U
(43) Date of publication of application: 08.11.2017
(73) Proprietor: Touchstone International Medical Science Co., Ltd., Suzhou, Jiangsu 215123 (CN)
(72) Inventor: CHEN, Wangdong, Suzhou Jiangsu 215021 (CN); SHU, Tuo, Suzhou Jiangsu 215021 (CN); FU, Kaifen, Suzhou Jiangsu 215021 (CN); PEI, Yongwang, Suzhou Jiangsu 215021 (CN)
(74) Representative: Schrell, Andreas
(86) International application number: PCT/CN2015/099936
(87) International publication number: WO 2016/107585

(56) References cited:
- EP-A1- 2 777 530
- EP-A2- 1 782 738
- EP-A2- 2 653 113
- EP-A2- 2 777 532
- WO-A1-03/094746
- CA-A1- 2 795 323
- CN-A- 1 957 854
- CN-A- 102 743 201
- CN-A- 103 860 225
- CN-A- 103 860 225
- CN-U- 204 364 049
- US-A- 5 485 947
- US-A1- 2005 173 490
- US-A1- 2008 179 375

## Description

### Technical Field

The invention relates to the field of medical instruments, particularly, to a stapling head assembly with a lockout mechanism preventing from being fired for a second time and a suturing and cutting apparatus for endoscopic surgery with the stapling head assembly.

### Background

A suturing and cutting apparatus for surgery has been widely used for suturing wounds, suturing and cutting internal tissues. The surgery having developed so far has an increasing tendency to be minimally invasive surgery. Generally speaking, minimally invasive surgery means all operations which can decrease wounds; narrowly speaking, minimally invasive surgery means the operations processed under endoscope. Generally, a few small holes are needed to be opened on the patient's body for endoscopic surgery, so the cutting and suturing instruments and assistant instruments can be used for operation by going into the patient's body through the small holes. With the little wounds caused from the minimally invasive surgery, the patients can recover in a very short time, so the minimally invasive surgery has gotten more and more attention.

The linear suturing and cutting apparatus for endoscopic surgery used in minimally invasive surgery in the existing technology, comprise an instrument body, said instrument body comprises a sleeve and an actuating handle pivoted to the sleeve, there is an actuating rod removable set in the sleeve, said stapling head assembly at the front end of the sleeve can be pushed by the actuating rod to process suturing and cutting. Specifically, said stapling head assembly comprises a cartridge support, and an anvil pivotally connected to the cartridge support. There is a cartridge detachably set on the cartridge support; there is a group of staple holes accommodating staple pushers and staples inside, generally 4 rows or 6 rows of staple holes. There are grooves for staple deformation set on said anvil relative to said staple holes. Said stapling head assembly further comprises a cutter pushing rod which is set movably in a connecting tube of the stapling head assembly and connects with the actuating rod, there is an I-shaped cutter at the distal end of the cutter pushing rod, the cartridge and the anvil move close to each other during the advancing process of the I-shaped cutter. A blade is set at the distal end of the cutter, and during its advancing process, the tissues between the cartridge and anvil are cut off. There is an actuating block in the cartridge, during the advancing process of the I-shaped cutter, the staple pushers are pushed in a sequence in the cartridge by the actuating block under the actuation of the I-shaped cutter, then the staples are pushed out of the cartridge by the staple pushers and staple the tissues. In the existing technology, after the above action of suturing and cutting being finished by the doctor, the instrument is needed to be reset, and then the cartridge is needed to be changed, so the suturing and cutting apparatus for endoscopic surgery can be used for the next suturing and cutting operation.

But some careless doctors or some doctors with lack of experience, may process the next suturing and cutting operation without changing the cartridge which has been fired, which will lead to a medical negligence, that is, cutting is processed without suturing. To prevent from this kind of negligence, a theory of preventing from being fired for a second time using a lockout mechanism is raised, that is, the actuating handle cannot be pressed without changing the cartridge which has been fired. Then, it prevents fundamentally from the medical negligence caused by the incorrect operation of the doctor.

Each of the patent application documents EP1782738A2, CN103860225A, EP2777532A2, EP2777530A1, CA2795323A1, EP2653113A2, WO03094746A1 and US2005173490A1, discloses a suturing and cutting apparatus. However, the lockout mechanisms of the references are in complex structure, and will decrease the users' experience.

### Summary

The invention provides a stapling head assembly and a suturing and cutting apparatus for endoscopic surgery, which can prevent the suturing and cutting apparatus from being fired for a second time without changing the fired cartridge.

One aspect of the invention provides a stapling head assembly, and said stapling head assembly comprises: a cartridge support, equipped detachably with a cartridge and comprising a first limiting groove along the longitudinal direction thereof; an actuating block with an unlocking component, set movably in said cartridge; a cutter, movable at least between a first position and a second position along the longitudinal direction of said cartridge support, wherein, when said cutter moves from the first position distally to the second position, said actuating block is pushed to move by said cutter, so that said cutter and said actuating block move along said first limiting groove; two lockout mechanisms at two sides of said first limiting groove, wherein each lockout mechanism comprises: a lockout block, set in said cartridge support, and movable between a third position and a fourth position along the transversal direction of said cartridge support relative to said cartridge support; a flexible component, set between said cartridge support and said lockout block, and actuating said lockout block to move from said third position to said fourth position towards said first limiting groove; before said actuating block being actuated to move by said cutter, and when said cutter is located at the first position proximal to the lockout blocks, said unlocking component squeezes said lockout blocks transversally to be at said third position, and keeps said flexible components under compression status, such that said cutter can move from said first position to said second position to actuate the actuating block; after the actuating block being actuated to move by said cutter, when said actuating block remains at a distal end of the cartridge, and when said cutter is reset to the first position, said lockout blocks are moved to said fourth position under the action of said flexible components, such that the space between said lockout blocks is smaller than the transversal thickness of said cutter and said cutter is prevented from moving from said first position to said second position by said lockout blocks.

In a preferable embodiment of the invention, said cartridge support further comprises two second limiting grooves along the transversal direction thereof, said second limiting grooves communicate with said first limiting groove, said lockout mechanisms are set in said second limiting grooves, and said lockout blocks are configured to move in said second limiting grooves between said third positions said fourth positions.

In a preferable embodiment of the invention, after said actuating block being actuated to move by said cutter, each lockout block extends into said first limiting groove under the action of said flexible component, and is in said fourth position, between the first position and the second position of said cutter, and said cutter is located at the distal end of each lockout block.

In a preferable embodiment of the invention, each flexible component is a flexible block, said flexible block is farther away from said first limiting groove than each lockout block, and one end thereof connects to the inner wall of said cartridge support, the other end connects to each lockout block.

In a preferable embodiment of the invention, said unlocking component is a bump set protruded at the bottom of the proximal end of said actuating block.

In a preferable embodiment of the invention, each lockout block further comprises a first guiding portion, such that, when said cutter moves from said second position to said first position, the lockout blocks are guided to move from said fourth position towards the direction of the third position.

In a preferable embodiment of the invention, said first guiding portion is a first slope, said first slope is set at the distal end of each lockout block, and inclines protruded from the distal end of each lockout block towards the direction of said first limiting groove.

In a preferable embodiment of the invention, each lockout block comprises a third guiding portion, such that, when said cartridge is being mounted, said lockout blocks are guided to move from said fourth position towards the direction of the third position.

In a preferable embodiment of the invention, said third guiding portion is a third slope, said third slope is set on the top surface of each lockout block, and inclines protruded from the top surface of each lockout block towards the direction of the first limiting groove.

In a preferable embodiment of the invention, said lockout mechanism further comprises a fixing cover, each lockout block and each flexible component are covered between said fixing cover and said cartridge support.

In a preferable embodiment of the invention, said fixing cover for lockout mechanism has a concave portion, each lockout block is further equipped with a protruded portion, said protruded portion is set on the top surface of said lockout block, and extends accordingly into said concave portion.

Another aspect of the invention provides a suturing and cutting apparatus for endoscopic surgery, comprising the stapling head assembly above.

With the stapling head assembly and the suturing and cutting apparatus for endoscopic surgery of the invention, a next operation by the doctor without changing a cartridge having been fired is prevented effectively, and medical negligence is avoided, and the structure of the suturing and cutting apparatus is simple and effective.

### Brief description of the figures

By reading of the detailed description of the nonrestrictive embodiments referring to the figures following, the other technical features, objective and advantages will be more apparent:
Fig. 1 is a stereoscopic view of the stapling head assembly of the invention;
Fig. 2a is an explosive view of the stapling head assembly of the invention;
Fig. 2b is front view of the actuating block of the stapling head assembly of the invention;
Fig. 3 is a structural schematic view of the longitudinal section of the stapling head assembly before being actuated;
Fig. 4 is a structural schematic view of the A-A section of Fig. 3 before the stapling head assembly being actuated;
Fig. 5 is a structural schematic view of the B-B section of Fig. 3 before the stapling head assembly being actuated;
Fig. 6 is a structural schematic view of the A-A section of Fig. 3 during the stapling head assembly being actuated;
Fig. 7 is a structural schematic view of the B-B section of Fig.3 during the stapling head assembly being actuated;
Fig. 8a is a structural schematic view of the B-B section of Fig. 3 during the reset process of the cutter after the stapling head assembly being actuated;
Fig. 8b is a locally enlarged schematic view of the position D of Fig. 8a; and
Fig. 9 is a structural schematic view of the B-B section of Fig. 3 after the reset process of the cutter of the stapling head assembly.

### Detailed description

The descriptions of the proximal end and the distal end, all refer to the operator of the instrument, the proximal end is the end close to the operator, and the distal end is the end far from the operator. The longitudinal direction of the invention means the direction of length of the component, generally the direction from the distal end to the proximal end. The transversal direction means the direction vertical to the longitudinal direction. The descriptions of direction or position like top/ up, bottom/down, vertical, horizontal and so on are descriptions using the figures as an example, and can be changed according to different requirements. Besides, the terms first, second, third, fourth and so on are only for description, cannot be understood to indicate or imply relative significance or imply the numbers of the technical features indicated.

According to the conception of the purpose, the stapling head assembly comprises a cartridge support, an anvil, a cartridge, an actuating block, a cutter pushing rod, a cutter and a lockout mechanism. There is a cartridge detachably set on said cartridge support; the anvil can pivot and move relatively close to the cartridge; the actuating block is set movably inside the cartridge, there is an unlocking component set on said actuating block; said cutter can at least move between a first position and a second position relative to said cartridge support and along the longitudinal direction thereof, there is a cutter pushing rod at the proximal end of said cutter, there is a blade at the distal end of said cutter, under the push of the cutter pushing rod, said actuating block is pushed to move by said cutter; said lockout mechanism comprises: a lockout block, said lockout block is set inside the cartridge support, and can move between a third position and a fourth position along the longitudinal direction of said cartridge support relative to said cartridge support; a flexible component, is set between said cartridge and said lockout block, actuating the lockout block to move from said third position to said fourth position; before said actuating block being actuated to move by said cutter, said unlocking component squeezes said lockout block transversally to make it be at the third position, said flexible portion is under compression status, said cutter can move from said first position to said second position; after said actuating block being actuated to move by said cutter, said lockout block moves to said fourth position under the action of said flexible component, said cutter is prevented from moving from said first position to said second position by said lockout block.

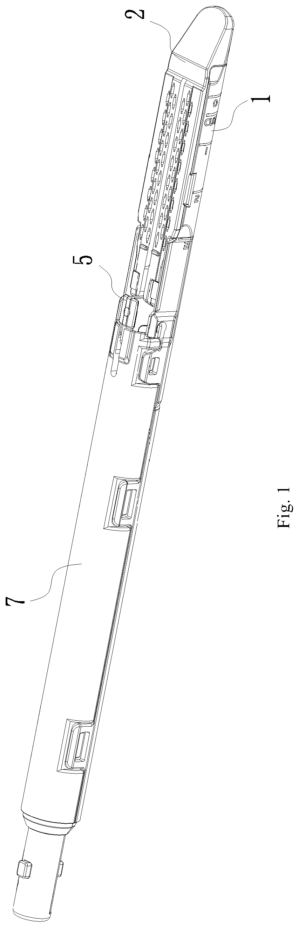

Below the technical contents of the invention are further illustrated combining the figures and embodiments:
Please refer to Fig. 1~ Fig. 5 together, which show separately a stereoscopic view, an explosive view of the stapling head assembly and structural schematic views of sections of the stapling head assembly before being actuated. Wherein, as the anvil of the stapling head assembly is not a part of the innovations of the invention, so, to show the structure of the stapling head assembly more clearly, the anvil of the stapling head assembly is ignored in the figures. In a preferable embodiment of the invention, the stapling head assembly comprises: a cartridge support 1, an anvil (not shown), a cartridge 2, an actuating block 3, a cutter pushing rod 4, a cutter 5, a connecting tube 7 and a lockout mechanism. Wherein, said anvil connects to the top of the cartridge support 1, preferably, the proximal end of the anvil pivots with the proximal end 11 of the cartridge support 1.

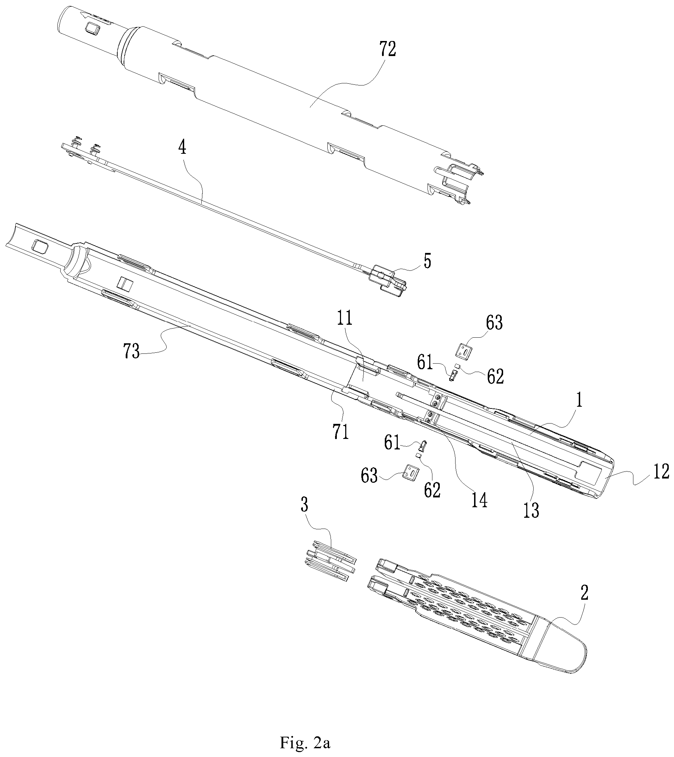

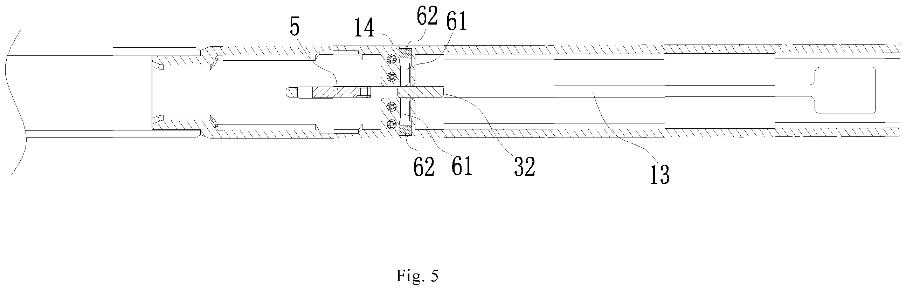

As shown in Fig. 2a and Fig. 5, there is a first limiting groove (cutting groove) 13 and a second limiting groove 14 on the cartridge support 1. The first limiting groove 13 is set along the direction from the proximal end 11 to the distal end 12 of the cartridge support 1. The second limiting groove 14 is set at a side portion of the first limiting groove 13 and communicates with the first limiting groove 13. In the preferable embodiment shown in Fig. 2a, a second limiting groove 14 is set on each of the two sides of the first limiting groove 13, and the two of the second limiting grooves 14 are in symmetry with each other relative to the first limiting groove 13.

The cartridge 2 is set between the cartridge support 1 and said anvil, and connects detachably to the cartridge support 1. More specifically, there are staple holes in the cartridge 2 for accommodating the staple pushers and staples, generally there are 4 rows or 6 rows of staple holes, there are grooves for staple deformation set on said anvil relative to said staple holes of the cartridge, by which suturing can be operated.

The distal end 71 of the connecting tube 7 connects to the proximal end 11 of the cartridge support 1. As shown in Fig. 2a, preferably, the connecting tube 7 is formed with an upper connecting component 72 and a lower connecting component 73 joining with each other.

The cutter pushing rod 4 is set movably in the connecting tube 7, the distal end 41 of the cutter pushing rod 4 is connected with a cutter 5. The distal end 52 of the cutter 5 is set with a blade. During the process of the cutter 5 being pushed to move towards the distal end by said cutter pushing rod 4, the tissues between the anvil and the cartridge 2 can be cut off by the blade, meanwhile, the actuating block 3 can be pushed to move by the cutter 5, so the staple pushers are pushed out of the cartridge 2 in sequence, then the staples are pushed out of the cartridge 2 and stapled on the tissues. In the embodiment, the longitudinal section of the cutter 5 is in a shape of "I", during the process of the cutter 5 being pushed to move towards the distal end by said cutter pushing rod 4, the I-shaped cutter 5 drives the anvil and the cartridge 2 to move close to each other.

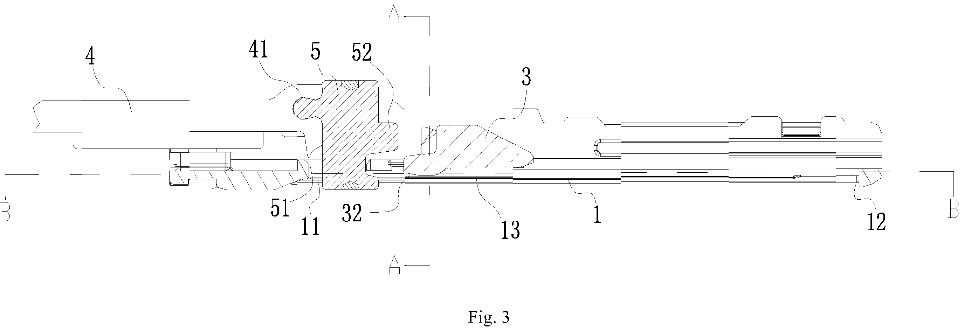

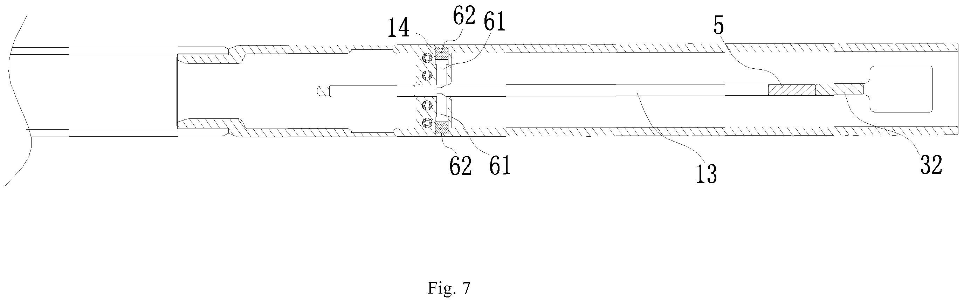

As shown in Fig. 3 and Fig. 5, the cutter 5 moves along the first limiting groove 13, the cutter 5 can at least move from the first position to the second position relative to the cartridge support 1 along the longitudinal direction thereof, the first position and the second position are two positions along the longitudinal direction of the cartridge support 1, and the first position is closer to the proximal end 11 of the cartridge support 1 than the second position. The position of the cutter 5 shown in Fig. 5 is the first position, that is, after the cartridge 2 being mounted on the cartridge support 1, the cutter 5 is set at the initial position of proximal end of the cartridge support 1; the position of the cutter 5 shown in Fig. 7 is the second position, that is, the position when the cutter 5 moves to the distal end of the cartridge support 1 under the action of the cutter pushing rod 4 and cannot move further towards the distal end.

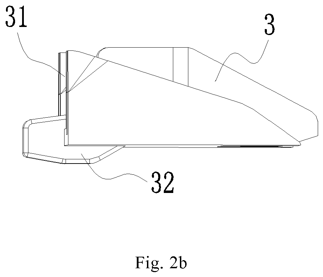

The actuating block 3 is set movably in the cartridge 2. The actuating block 3 comprises an unlocking component. Said unlocking component is set in the first limiting groove 13. As shown in Fig. 2b, said unlocking component is a bump 32 set protruded at the bottom of the proximal end 31 of the actuating block 3. The specific function of the bump 32 can be seen as below.

The lockout mechanism is set in the second limiting groove 14; said lockout mechanism comprises a lockout block 61 and a flexible component.

The lockout block 61 is set in the cartridge support 1, and movable between the third position and the fourth position relative to the cartridge support 1, when the lockout block 61 is set at said third position, the cutter 5 can move from said first position to said second position, when the lockout block 61 is located at said fourth position, the cutter 5 is prevented from moving from said first position to said second position by the lockout block 61.

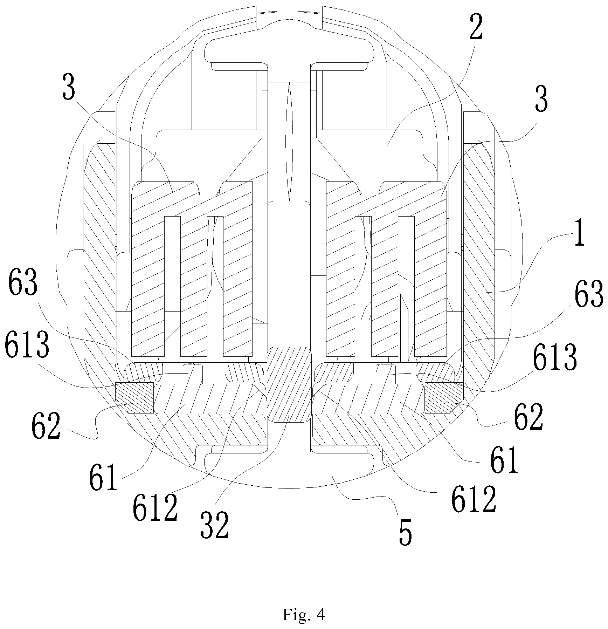

More specifically, there is a third guiding portion at the top surface of the lockout block 61, in the embodiment, the third guiding portion is an inclined third slope 612 set on the top surface of the lockout block 61, and inclines and protrudes along the direction from the top surface of the lockout block 61 to the first limiting groove 13. The third slope 612 cooperates with the bump 32 of the actuating block 3, to make it easier for inserting the actuating block 3 during the cartridge 2 being mounted on the cartridge support 1. The lockout block 61 moves in the second limiting groove 14, said fourth position is closer to the first limiting groove 13 than said third position, the position of the lockout block 61 shown in Fig. 4 and Fig. 5 is the third position, that is, during the process of the cartridge 2 being mounted on the cartridge support 1, the bump 32 of the actuating block 3 set at the proximal end of the cartridge 2 squeezes transversally the lockout block 61 along the third slope 612, then the flexible component is squeezed by the lockout block 61 to happen a flexible deformation, and eventually the lockout block 61 is away from the moving path of the cutter 5 from the first position to the second position, the position now of the lockout block 61 is the third position; the position of the lockout block 61 shown in Fig. 7 is the fourth position, specifically, during the process of the cutter 5 being driven by the pushing of the cutter pushing rod 4 from the first position to the second position, the actuating block 3 is pushed to move towards the distal end of the cartridge 2 by the cutter 5 at the same time, so the squeezing action from the actuating block 3 to the lockout block 61 is decreased gradually, when the actuating block 3 and the cutter 5 keep moving towards the distal end until they won't have interactions with the lockout block 61, the lockout block 61 won't be limited at all, and moves along the second limiting groove 14 under the action of the flexible component, and part of the lockout block 61 enters into the first limiting groove 13, so the lockout block 61 is a block on the moving path of the cutter 5 between the first position and the second position. As shown in Fig. 4 and Fig. 5, preferably, said stapling head assembly comprises two lockout mechanisms, set separately in the two second limiting grooves 14 at the two sides of the first limiting groove 13.

The flexible component is set in the cartridge support 1, and drives the lockout block 61 to move from the third position to the fourth position. As shown in Fig. 5, the flexible component is farther away from the first limiting groove 13 than the lockout block 61, one end of the flexible component connects to the second limiting groove 14, and the other end connects to the lockout block 61. The flexible component is preferably a flexible block 62. The flexible block 62 is set in the second limiting groove 14, one end of the flexible block 62 connects to the inner wall of the cartridge support 1, and the other end connects to the lockout block 61. The technicians in the art should understand that, in some embodiments, the flexible component can be different, such as, the flexible component can be a spring which is set between the second limiting groove 14 and the lockout block 61. No unnecessary details are given here.

In the preferable embodiment shown in Fig. 2a, the lockout mechanism further comprises a fixing cover 63 for lockout mechanism, the lockout block 61 and the flexible component (that is, the flexible block 62) are covered by the fixing cover 63 and set between the fixing cover 63 and the second limiting groove 14 of the cartridge support 1. There are holes set on the fixing cover 63, so the fixing cover 63 can be fixed in the second limiting groove 14 by fixing at least one bolt into the holes. Further, in the preferable embodiment shown in Fig. 4, there is a concave portion on the fixing cover 63, the lockout block 61 further comprises a protruded portion 613, and the protruded portion 613 is set on the top surface of the lockout block 61, the concave portion is set on the lower bottom of the fixing cover 63 relative to the protruded portion 613. The protruded portion 613 extends upwards into the concave portion of the fixing cover 63, the protruded portion 613 can effectively prevent from the condition that the third slope 612 is installed reversely if the lockout block 61 is assembled reversely, then the third slope 612 will lose the guiding function, result in that the cartridge 2 could not be mounted in place smoothly.

Further, please refer to Fig. 4 and Fig. 5, after the cartridge 2 being mounted on the cartridge support 1 and before the stapling head assembly being actuated, the cutter 5 is located at the first position, the unlocking component (that is the bump 32) of the actuating block 3 at the proximal end of the cartridge 2 squeezes the lockout block 61 transversally to move to the third position, the lockout block 61 is restricted by the bump 32, so the flexible component (that is the flexible block 62) is under compression status, at the moment, the cutter pushing rod 4 can be pushed to actuate the cutter 5 and the actuating block 3 to move towards the distal end, so the tissue can be cut and sutured.

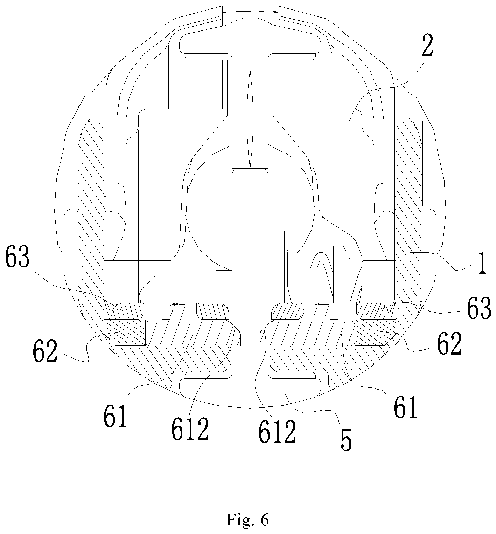

Furthermore, please refer to Fig. 6~ Fig. 9 together.

Fig. 6 and Fig. 7 show separately the structural schematic views of the A-A section and B-B section during the stapling assembly of Fig. 3 being actuated. As shown in Fig. 6 and Fig. 7, after the actuating block 3 being pushed by the cutter 5 under the action of the cutter pushing rod 4, the actuating block 3 moves towards the distal end 12 of the cartridge support 1 along a first limiting groove 13 and departs away from the lockout block 61. During the process, the cutter 5 moves from the first position to the second position, the lockout block 61 will not be squeezed transversally for that the unlocking component (that is the bump 32) of the actuating block 3 is moved, and then moves to a fourth position (such as the lockout blocks 61 at the two sides are closed to each other, the space between the two lockout blocks 61 is smaller than the transversal thickness of the cutter 5) under the flexible action of the flexible component (flexible block 62). The position of the cutter 5 shown in Fig. 7 is the second position. Meanwhile, the position of the lockout block 61 shown in Fig. 6 and Fig. 7 is the fourth position. As shown in Fig. 7, when the lockout block 61 is at the fourth position, part of the lockout block 61 extends into the first limiting groove 13, and is located between the first position and the second position of the cutter 5.

Further, for that in the preferable embodiment of the invention, said stapling head assembly comprises two lockout mechanisms set at the two sides of the first limiting groove 13. Hence, in the embodiment, the lockout block 61 being at the fourth position means that the two lockout blocks 61 being all set at the fourth position, that is, partial structures of the two lockout blocks 61 all extend into the first limiting groove 13 and are located between said first position and the second position. As shown in Fig. 6, when the two lockout blocks 61 are all set at the fourth position, the space between the two lockout blocks 61 is smaller than the transversal thickness of the cutter 5.

Preferably, the transversal thickness of the cutter 5 is smaller than or equal to the transversal thickness of the bump 32 of the actuating block 3, which is more beneficial when the bump 32 of the actuating block 3 moves and the cutter 5 can be moved from the first position to the second position before the lockout block 61 moving from the third position to the fourth position.

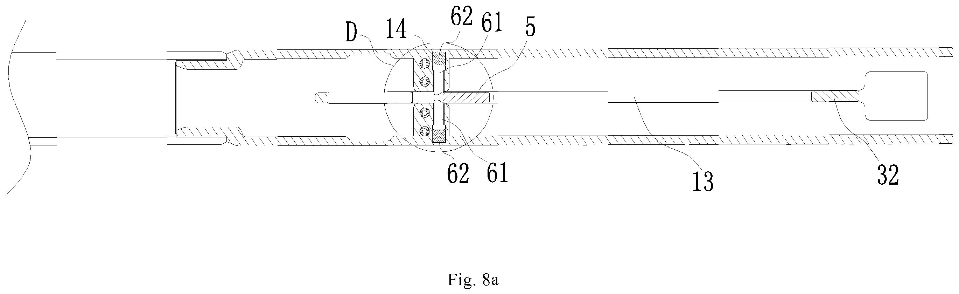

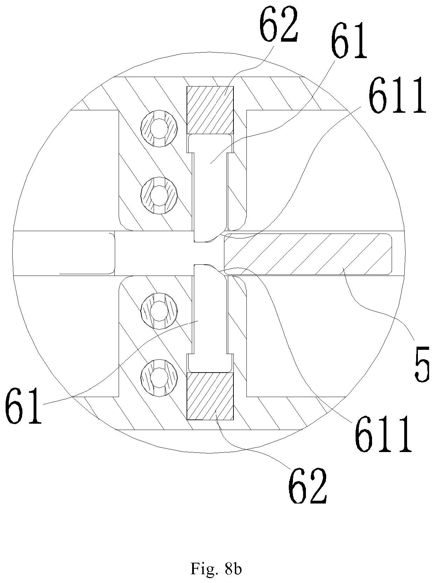

Further, when the cutting and suturing of the tissues are finished, the cutter 5 is needed to be reset to go back to the first position before being actuated, the actuating block 3 stays at the distal end of the cartridge. Fig. 8a and Fig. 8b show the structural schematic view of the B-B section during the reset process of the cutter 5 after the stapling head assembly being fired; Fig. 8b is a locally enlarged schematic view of the position D of Fig. 8a. As shown in Fig. 8a and 8b, the lockout block 61 comprises a first guiding portion, the first guiding portion is used to guide the lockout block 61 to move from the fourth position towards the direction of the third position during the cutter 5 moving from the second position to the first position. In the embodiment shown in Fig. 8b, said first guiding portion is a first slope 611, the first slope 611 is set at the distal end of the lockout block 61, the first slope 611 inclines protruded from the distal end of the lockout block 61 towards the direction of the first limiting groove 13, for example the section of the lockout block 61 can be in a shape of trapezoid, and extends from the distal end of the lockout block 61 to one side of the lockout block 61 close to the first limiting groove 13. When the cutter 5 moves from the second position towards the direction of the first position to the distal position of the lockout block 61, the first slope 611 is pushed by the cutter 5, the lockout block 61 is made to move towards the direction of the third position by the component force along the direction of the second limiting groove 14 caused by the first slope 611, the flexible component (that is the flexible block 62) is under the compression status again. When the lockout blocks 61 are totally located at the two sides of the cutter 5, the lockout blocks 61 are at a fifth position. It should be understood that, the transversal thickness of the cutter 5 is less than or equal to the transversal thickness of the bump 32 of the actuating block 3, hence, the fifth position is located between the third position and the fourth position. When the transversal thickness of the cutter 5 is the same with the transversal thickness of the bump 32, the fifth position is a same position with the third position.

Further, preferably, the proximal end 51 of the cutter 5 also comprises a second guiding portion, said second portion cooperates with the first guiding portion, and guides the lockout block 61 to move from the fourth position towards the direction of the third position. Said second guiding portion can be at least one second slope adapted to the first slope 611. Said second slopes are set accordingly at the two sides of the proximal end 51 of the cutter 5. The setting of the second slopes is more beneficial for the proximal end 51 of the cutter 5 to squeeze transversally the lockout blocks 61 to the third position.

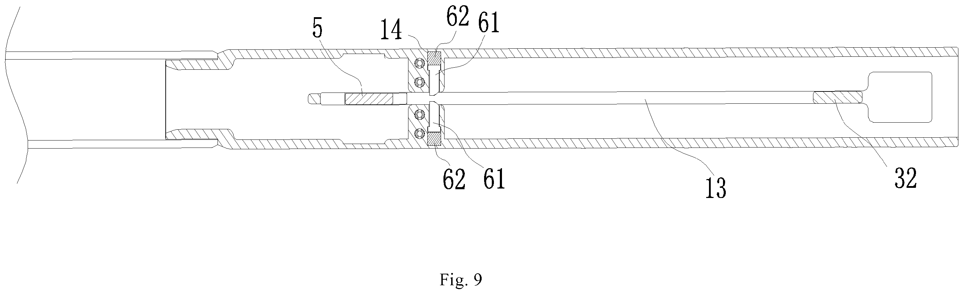

Fig. 9 shows the structural schematic of the B-B section after the cutter being reset. As shown in Fig. 9, during the process of the cutter 5 going back to the first position, when the cutter 5 moves to a position that it won't have interaction force with the lockout block 61, there won't be any transversal squeezing from the cutter 5 between the two lockout blocks 61, the lockout blocks 61 move again to the fourth position under the flexible action of the flexible component (flexible block 62), and block the cutter between said first position and said second position. As the space between the two lockout blocks 61 is smaller than the transversal thickness of the cutter 5, and there is no guiding portion at the proximal end of the lockout blocks 61, so, if a second actuating is processed, the lockout blocks 61 will prevent the cutter 5 from moving from the first position to the second position, and the instrument won't be fired for a second time, and can only be fired normally after a new cartridge being changed. And when a new cartridge is changed, the bump 32 of the actuating block of the new cartridge can be squeezed between the two lockout blocks 61 from the top of the space between the two lockout blocks 61 by the guiding of the third slopes 612, and the lockout blocks 61 are forced to go back to the third position again, that is going back to the status shown in Fig. 5. Hence, if a new cartridge 2 is not changed, the lockout blocks 61 cannot be squeezed transversally into the second limiting groove 14, and the cutter 5 is blocked by the lockout blocks 61, and cannot move towards the second position again, so the function of preventing the stapling head assembly from being fired for a second time without changing the fired cartridge is realized.

The structure of preventing from being fired for a second time of the embodiment mainly uses the action force of the flexible components (flexible blocks 62) set at the two sides of the first limiting groove 13 to the lockout block 61, after the lockout blocks 61 losing the transversal squeezing of the unlocking component (bump 32) of the actuating block 3, the lockout blocks 61 on two sides come closer with each other, so the firing for a second time after the cutter 5 being pulled back is prevented.

The technicians in the art should understand that, in different embodiments of the invention, the function of preventing the stapling head assembly from being fired for a second time can also be realized by one said lockout mechanism. In the alternative embodiment, there is a second limiting groove 14 set at one side of the first limiting groove 13, the lockout mechanism is set in the second limiting groove 14, when the lockout block 61 is set at the fourth position, the lockout block 61 protrudes from the second limiting groove 14 towards the direction of the inner wall of one corresponding side of the first limiting groove 13, and the protruding portion of the lockout block 61 is located at the moving path of the cutter 5 moving from the first position to the second position, so the cutter 5 is restricted from moving towards the distal end, at the moment, the function of preventing from being fired for a second time can also be realized by one said lockout mechanism, which will not be repeated here.

The invention further provides a suturing and cutting apparatus for endoscopic surgery. The suturing and cutting apparatus comprises a body and the stapling head assembly. Wherein, the body comprises a sleeve and an actuating handle set in the sleeve, the stapling head assembly is the stapling head assembly shown in Fig. 1~Fig. 9. The connecting tube 7 and the cutter pushing rod 4 of the stapling head assembly connect to the body. The actuating handle can push the cutter pushing rod 4, so the cutter 5 is made to move towards the distal end 12 of the cartridge support 1.

Combining with the embodiment shown in Fig. 1~ Fig. 9, the technicians in the art should understand that, with the stapling head assembly and the suturing and cutting apparatus for endoscopic surgery of the invention, a next operation by the doctor without changing a cartridge having been fired is prevented effectively, medical negligence is avoided, and the structure of the suturing and cutting apparatus is simple and effective.

The specific embodiments of the invention are described above. It should be understood that, the invention is not limited to the specific embodiments above; the technicians in the art can make all kinds of transformation and amendments within the scope of the claims, which will not affect the substantial contents of the invention.

## Claims

1. A stapling head assembly, and said stapling head assembly comprises:
a cartridge support (1), equipped detachably with a cartridge (2) and comprising a first limiting groove (13) along the longitudinal direction thereof;
an actuating block (3) with an unlocking component (32), set movably in said cartridge;
a cutter (5), movable at least between a first position and a second position along the longitudinal direction of said cartridge support (1), wherein, when said cutter (5) moves from the first position distally to the second position, said actuating block (3) is pushed to move by said cutter (5), so that said cutter (5) and said actuating block (3) move along said first limiting groove (13);
two lockout mechanisms at two sides of said first limiting groove (13), wherein each lockout mechanism comprises:
a lockout block (61), set in said cartridge support (1), and movable between a third position and a fourth position along the transversal direction of said cartridge support (1) relative to said cartridge support (1);
a flexible component (62), set between said cartridge support (1) and said lockout block (61), and actuating said lockout block (61) to move from said third position to said fourth position towards said first limiting groove (13);
before said actuating block (3) being actuated to move by said cutter (5), and when said cutter (5) is located at the first position proximal to the lockout blocks (61), said unlocking component (32) squeezes said lockout blocks (61) transversally to be at said third position, and keeps said flexible components (62) under compression status, such that said cutter (5) can move from said first position to said second position to actuate the actuating block (3);
after the actuating block (3) being actuated to move by said cutter (5), when said actuating block (3) remains at a distal end of the cartridge (2), and when said cutter (5) is reset to the first position, said lockout blocks (61) are moved to said fourth position under the action of said flexible components (62), such that the space between said lockout blocks (61) is smaller than the transversal thickness of said cutter (5) and said cutter (5) is prevented from moving from said first position to said second position by said lockout blocks (61).

2. The stapling head assembly according to claim 1, wherein, said cartridge support (1) further comprises two second limiting grooves (14) along the transversal direction thereof, said second limiting grooves (14) communicate with said first limiting groove (13), said lockout mechanisms are set in said second limiting grooves (14), and said lockout blocks (61) are configured to move in said second limiting grooves (14) between said third positions and said fourth positions.

3. The stapling head assembly according to claim 2, wherein, after said actuating block (3) being actuated to move by said cutter (5), each lockout block (61) extends into said first limiting groove (13) under the action of said flexible component (62), and is in said fourth position, between the first position and the second position of said cutter (5), and said cutter (5) is located at the distal end of each lockout block (61).

4. The stapling head assembly according to any item of claims 1-3, wherein, each flexible component (62) is a flexible block, said flexible block is farther away from said first limiting groove (13) than each lockout block (61), and one end thereof connects to the inner wall of said cartridge support (1), the other end connects to each lockout block (61).

5. The stapling head assembly according to any item of claims 1-3, wherein, said unlocking component (32) is a bump (32) set protruded at the bottom of the proximal end of said actuating block (3).

6. The stapling head assembly according to claim 1, wherein, each lockout block (61) further comprises a first guiding portion, such that, when said cutter (5) moves from said second position to said first position, the lockout blocks (61) are guided to move from said fourth position towards the direction of the third position.

7. The stapling head assembly according to claim 6, wherein, said first guiding portion is a first slope (611), said first slope (611) is set at the distal end of each lockout block (61), and inclines protruded from the distal end of each lockout block (61) towards the direction of said first limiting groove (13).

8. The stapling head assembly according to claim 1, wherein, each lockout block (61) comprises a third guiding portion, such that, when said cartridge (2) is being mounted, said lockout blocks (61) are guided to move from said fourth position towards the direction of the third position.

9. The stapling head assembly according to claim 8, wherein, said third guiding portion is a third slope (612), said third slope (612) is set on the top surface of each lockout block (61), and inclines protruded from the top surface of each lockout block (61) towards the direction of the first limiting groove (13).

10. The stapling head assembly according to claim 1, wherein, each lockout mechanism further comprises a fixing cover (63), each lockout block (61) and each flexible component (62) are covered between said fixing cover (63) and said cartridge support (1).

11. The stapling head assembly according to claim 10, wherein, said fixing cover (63) for lockout mechanism comprises a concave portion, each lockout block (61) is further equipped with a protruded portion (613), said protruded portion (613) is set on the top surface of said lockout block (61), and extends accordingly into said concave portion.

12. A suturing and cutting apparatus for endoscopic surgery, comprising the stapling head assembly of any one of claims 1-11.

## Patentansprüche

1. Klammerkopfanordnung, und wobei die Klammerkopfanordnung umfasst:
einen Magazinhalter (1), der abnehmbar mit einem Magazin (2) ausgestattet ist und eine erste Begrenzungsnut (13) in seiner Längsrichtung umfasst;
einen Betätigungsblock (3) mit einer Entriegelungskomponente (32), der beweglich in dem Magazin angeordnet ist; eine Schneidevorrichtung (5), das zumindest zwischen einer ersten Position und einer zweiten Position entlang der Längsrichtung des Magazinhalters (1) beweglich ist, wobei, wenn sich die Schneidevorrichtung (5) von der ersten Position in distaler Richtung in die zweite Position bewegt, der Betätigungsblock (3) von der Schneidevorrichtung (5) in Bewegung versetzt wird, so dass sich die Schneidevorrichtung (5) und der Betätigungsblock (3) entlang der ersten Begrenzungsnut (13) bewegen;
zwei Verriegelungsmechanismen an zwei Seiten der ersten Begrenzungsnut (13), wobei jeder Verriegelungsmechanismus umfasst:
einen Verriegelungsblock (61), der in den Magazinhalter (1) eingesetzt ist und zwischen einer dritten Position und einer vierten Position entlang der Querrichtung des Magazinhalters (1) relativ zum Magazinhalter (1) beweglich ist;
ein flexibles Bauteil (62), das zwischen dem Magazinhalter (1) und dem Verriegelungsblock (61) angeordnet ist und den Verriegelungsblock (61) betätigt, um sich von der dritten Position in die vierte Position in Richtung der ersten Begrenzungsnut (13) zu bewegen;
bevor der Betätigungsblock (3) durch die Schneidevorrichtung (5) zur Bewegung betätigt wird, und wenn sich die Schneidevorrichtung (5) in der ersten Position proximal zu den Verriegelungsblöcken (61) befindet, drückt die Entriegelungskomponente (32) die Verriegelungsblöcke (61) in Querrichtung zusammen, um sich in der dritten Position zu befinden, und hält die flexiblen Bauteile (62) unter Druck, so dass sich die Schneidevorrichtung (5) von der ersten Position zur zweiten Position bewegen kann, um den Betätigungsblock (3) zu betätigen;
nachdem der Betätigungsblock (3) durch die Schneidevorrichtung (5) zur Bewegung betätigt wurde, wenn der Betätigungsblock (3) an einem distalen Ende des Magazins (2) verbleibt, und wenn die Schneidevorrichtung (5) in die erste Position zurückgestellt wird, werden die Verriegelungsblöcke (61) unter der Wirkung der flexiblen Bauteile (62) in die vierte Position bewegt, so dass der Raum zwischen den Verriegelungsblöcken (61) kleiner ist als die Querdicke der Schneidevorrichtung (5) und die Schneidevorrichtung (5) durch die Verriegelungsblöcke (61) daran gehindert wird, sich von der ersten Position in die zweite Position zu bewegen.

2. Klammerkopfanordnung gemäß Anspruch 1, wobei der Magazinhalter (1) ferner zwei zweite Begrenzungsnuten (14) entlang seiner Querrichtung umfasst, wobei die zweiten Begrenzungsnuten (14) mit der ersten Begrenzungsnut (13) in Verbindung stehen, die Verriegelungsmechanismen in die zweiten Begrenzungsnuten (14) eingesetzt sind und die Verriegelungsblöcke (61) so konfiguriert sind, dass sie sich in den zweiten Begrenzungsnuten (14) zwischen den dritten Positionen und den vierten Positionen bewegen.

3. Klammerkopfanordnung nach Anspruch 2, wobei sich nach der Betätigung des Betätigungsblocks (3) zur Bewegung durch die Schneidevorrichtung (5) jeder Verriegelungsblock (61) unter der Wirkung des flexiblen Bauteils (62) in die erste Begrenzungsnut (13) erstreckt und sich in der vierten Position zwischen der ersten Position und der zweiten Position der Schneidevorrichtung (5) befindet, und die Schneidevorrichtung (5) am distalen Ende jedes Verriegelungsblocks (61) angeordnet ist.

4. Klammerkopfanordnung nach einem der Ansprüche 1 bis 3, wobei jedes flexible Bauteil (62) ein flexibler Block ist, der flexible Block weiter von der ersten Begrenzungsnut (13) entfernt ist als jeder Verriegelungsblock (61) und ein Ende davon mit der Innenwand des Magazinhalters (1) verbunden ist und das andere Ende mit jedem Verriegelungsblock (61) verbunden ist.

5. Klammerkopfanordnung nach einem der Ansprüche 1 bis 3, wobei die Entriegelungskomponente (32) ein Vorsprung (32) ist, der an der Unterseite des proximalen Endes des Betätigungsblocks (3) vorsteht.

6. Klammerkopfanordnung nach Anspruch 1, wobei jeder Verriegelungsblock (61) ferner einen ersten Führungsabschnitt umfasst, so dass die Verriegelungsblöcke (61), wenn sich die Schneidevorrichtung (5) von der zweiten Position in die erste Position bewegt, geführt werden, um sich von der vierten Position in Richtung der dritten Position zu bewegen.

7. Klammerkopfanordnung nach Anspruch 6, wobei der erste Führungsabschnitt eine erste Schräge (611) ist, wobei die erste Schräge (611) am distalen Ende jedes Verriegelungsblocks (61) angeordnet ist und vom distalen Ende jedes Verriegelungsblocks (61) in Richtung der ersten Begrenzungsnut (13) vorsteht.

8. Klammerkopfanordnung nach Anspruch 1, wobei jeder Verriegelungsblock (61) einen dritten Führungsabschnitt umfasst, so dass die Verriegelungsblöcke (61) bei der Montage des Magazins (2) so geführt werden, dass sie sich von der vierten Position in Richtung der dritten Position bewegen.

9. Klammerkopfanordnung nach Anspruch 8, wobei der dritte Führungsabschnitt eine dritte Schräge (612) ist, wobei die dritte Schräge (612) auf der oberen Fläche jedes Verriegelungsblocks (61) angeordnet ist und von der oberen Fläche jedes Verriegelungsblocks (61) in Richtung der ersten Begrenzungsnut (13) vorsteht.

10. Klammerkopfanordnung gemäß Anspruch 1, wobei jeder Verriegelungsmechanismus ferner eine Befestigungsabdeckung (63) umfasst, wobei jeder Verriegelungsblock (61) und jedes flexible Bauteil (62) zwischen der Befestigungsabdeckung (63) und dem Magazinhalter (1) abgedeckt sind.

11. Klammerkopfanordnung nach Anspruch 10, wobei die Befestigungsabdeckung (63) für den Verriegelungsmechanismus einen konkaven Abschnitt umfasst, jeder Verriegelungsblock (61) ferner mit einem vorstehenden Abschnitt (613) ausgestattet ist, der vorstehende Abschnitt (613) auf der oberen Fläche des Verriegelungsblocks (61) angeordnet ist und sich entsprechend in den konkaven Abschnitt hinein erstreckt.

12. Näh- und Schneidevorrichtung für die endoskopische Chirurgie, umfassend die Klammerkopfanordnung nach einem der Ansprüche 1-11.

## Revendications

1. Un système de tête d'agrafage, et ledit système de tête d'agrafage comprend :
un support de cartouche (1), équipé de manière détachable d'une cartouche (2) et comprenant une première rainure de limitation (13) le long de sa direction longitudinale ;
un bloc de commande (3) avec un composant de déverrouillage (32), placé de manière mobile dans ladite cartouche ; un couteau (5), mobile au moins entre une première position et une seconde position le long de la direction longitudinale dudit support de cartouche (1), dans lequel, lorsque ledit couteau (5) se déplace de la première position distalement vers la seconde position, ledit bloc de commande (3) est poussé pour se déplacer par ledit couteau (5), de sorte que ledit couteau (5) et ledit bloc de commande (3) se déplacent le long de ladite première rainure de limitation (13) ;
deux mécanismes de verrouillage sur les deux côtés de ladite première rainure de limitation (13), dans lequel chaque mécanisme de verrouillage comprend :
un bloc de verrouillage (61), placé dans ledit support de cartouche (1), et mobile entre une troisième position et une quatrième position le long de la direction transversale dudit support de cartouche (1) par rapport audit support de cartouche (1) ;
un composant flexible (62), placé entre ledit support de cartouche (1) et ledit bloc de verrouillage (61), et commandant ledit bloc de verrouillage (61) pour se déplacer de ladite troisième position à ladite quatrième position vers ladite première rainure de limitation (13) ;
avant que ledit bloc de commande (3) ne soit commandé pour se déplacer par ledit couteau (5), et lorsque ledit couteau (5) est situé à la première position proximale aux blocs de verrouillage (61), ledit composant de déverrouillage (32) comprime lesdits blocs de verrouillage (61) transversalement pour être à ladite troisième position, et maintient lesdits composants flexibles (62) sous un état de compression, de sorte que ledit couteau (5) peut se déplacer de ladite première position à ladite seconde position pour commander le bloc de commande (3) ;
après que le bloc de commande (3) a été commandé pour se déplacer par ledit couteau (5), lorsque ledit bloc de commande (3) reste à une extrémité distale de la cartouche (2), et lorsque ledit couteau (5) est remis dans la première position, lesdits blocs de verrouillage (61) sont déplacés vers ladite quatrième position sous l'action desdits composants flexibles (62), de sorte que l'espace entre lesdits blocs de verrouillage (61) est plus petit que l'épaisseur transversale dudit couteau (5) et ledit couteau (5) est empêché de se déplacer de ladite première position à ladite seconde position par lesdits blocs de verrouillage (61).

2. Le système de tête d'agrafage selon la revendication 1, dans lequel, ledit support de cartouche (1) comprend en outre deux secondes rainures de limitation (14) le long de sa direction transversale, lesdites secondes rainures de limitation (14) communiquent avec ladite première rainure de limitation (13), lesdits mécanismes de verrouillage sont placés dans lesdites secondes rainures de limitation (14), et lesdits blocs de verrouillage (61) sont configurés pour se déplacer dans lesdites secondes rainures de limitation (14) entre lesdites troisièmes positions et lesdites quatrièmes positions.

3. Le système de tête d'agrafage selon la revendication 2, dans lequel, après que ledit bloc de commande (3) a été commandé pour se déplacer par ledit couteau (5), chaque bloc de verrouillage (61) s'étend dans ladite première rainure de limitation (13) sous l'action dudit composant flexible (62), et est dans ladite quatrième position, entre la première position et la deuxième position dudit couteau (5), et ledit couteau (5) est situé à l'extrémité distale de chaque bloc de verrouillage (61).

4. Le système de tête d'agrafage selon l'un quelconque des revendications 1-3, dans lequel, chaque composant flexible (62) est un bloc flexible, ledit bloc flexible est plus éloigné de ladite première rainure de limitation (13) que chaque bloc de verrouillage (61), et une extrémité de celui-ci se connecte à la paroi interne dudit support de cartouche (1), l'autre extrémité se connecte à chaque bloc de verrouillage (61).

5. Le système de tête d'agrafage selon l'un quelconque des revendications 1-3, dans lequel, ledit composant de déverrouillage (32) est une bosse (32) placée en saillie au fond de l'extrémité proximale dudit bloc de commande (3).

6. Le système de tête d'agrafage selon la revendication 1, dans lequel, chaque bloc de verrouillage (61) comprend en outre une première partie de guidage, de sorte que, lorsque ledit couteau (5) se déplace de ladite deuxième position à ladite première position, les blocs de verrouillage (61) sont guidés pour se déplacer de ladite quatrième position vers la direction de la troisième position.

7. Le système de tête d'agrafage selon la revendication 6, dans lequel, ladite première partie de guidage est une première inclinaison (611), ladite première inclinaison (611) est placée à l'extrémité distale de chaque bloc de verrouillage (61), et s'incline en saillie depuis l'extrémité distale de chaque bloc de verrouillage (61) vers la direction de ladite première rainure de limitation (13).

8. Le système de tête d'agrafage selon la revendication 1, dans lequel, chaque bloc de verrouillage (61) comprend une troisième partie de guidage, de sorte que, lorsque ladite cartouche (2) est montée, lesdits blocs de verrouillage (61) sont guidés pour se déplacer de ladite quatrième position vers la direction de la troisième position.

9. Le système de tête d'agrafage selon la revendication 8, dans lequel, ladite troisième partie de guidage est une troisième inclinaison (612), ladite troisième inclinaison (612) est placée sur la surface supérieure de chaque bloc de verrouillage (61), et s'incline en saillie depuis la surface supérieure de chaque bloc de verrouillage (61) vers la direction de la première rainure de limitation (13).

10. Le système de tête d'agrafage selon la revendication 1, dans lequel, chaque mécanisme de verrouillage comprend en outre un couvercle de fixation (63), chaque bloc de verrouillage (61) et chaque composant flexible (62) sont couverts entre ledit couvercle de fixation (63) et ledit support de cartouche (1).

11. Le système de tête d'agrafage selon la revendication 10, dans lequel, ledit couvercle de fixation (63) pour le mécanisme de verrouillage comprend une partie concave, chaque bloc de verrouillage (61) est en outre équipé d'une partie en saillie (613), ladite partie en saillie (613) est placée sur la surface supérieure dudit bloc de verrouillage (61), et s'étend en conséquence dans ladite partie concave.

12. Appareil de suture et de coupe pour la chirurgie endoscopique, comprenant le système de tête d'agrafage de l'une quelconque des revendications 1-11.
